# EUROPEAN PATENT SPECIFICATION

(11) **EP 3 049 146 B1**
(45) Date of publication and mention of the grant of the patent: **06.02.2019**
(21) Application number: 14777863.3
(22) Date of filing: 16.09.2014
(51) Int. Cl.: A61M 39/04, A61M 39/26, A61M 39/24

(54) **NEEDLE-FREE CONNECTOR**
NADELLOSER VERBINDER
RACCORD SANS AIGUILLE

(30) Priority: 25.09.2013 IT MO20130264
(43) Date of publication of application: 03.08.2016
(73) Proprietor: PIERC S.r.l., 41032 Cavezzo (MO) (IT)
(72) Inventor: MAFFEI, Giuseppe, 41037 Mirandola (MO) (IT)
(74) Representative: Brunacci, Marco
(86) International application number: PCT/IB2014/064563
(87) International publication number: WO 2015/044835

(56) References cited:
- WO-A1-97/21463
- WO-A1-2006/122406
- US-A- 5 676 346
- US-A1- 2009 209 922

## Description

### Technical Field

The present invention relates to a needle-free connector for infusion lines.

### Background Art

Needle-free connectors are known, used as valves for the selective insertion of fluids in infusion lines, e.g., of the type comprising venous catheters.

This type of connector permits the intravenous administration of drugs or other fluids using needle-free syringes or "sets" circuits terminating in a standard needle-free connector.

In detail, the syringes have a connection sleeve which fits into the connector through one of its free ends, moving a sealing element of the connector that seals such end when it is not coupled with syringes.

Such sleeve couples in a removable way to the above-mentioned free end, by means of a Luer connection or the like.

This way, the intravenous infusion of medical fluids can be obtained, avoiding cases of medical personnel being accidentally pricked or of accidental catheter lacerations.

Numerous cases have nevertheless occurred of blood infections related to the use of catheters (or CRBSI), the aetiology of which seems traceable to the use of this type of connector.

These infections are most likely tied to the formation of blood clots inside the catheter, due to a backflow of blood from the patient's vessel to the catheter connected to it, the backflow being caused by a phenomenon known in the technical sector by the name of *negative displacement,* i.e., the movement of the blood inside the catheter.

In fact, during the injection of the fluid from the syringe to the catheter, the above-mentioned sleeve occupies a certain volume inside the connector, where it is placed in fluid-dynamic communication with the catheter and, therefore, with the blood vessel.

Following the separation of the syringe from the connector itself, the sleeve leaves a space that tends to be filled by the fluid inside the catheter.

Such movement of the fluid from the catheter into the connector produces a backflow, or movement, of the patient's blood inside the catheter.

This blood can cause a clot which, in certain conditions, above all if of large size, can lead to the growth of pathogens and/or occlusions inside the catheter which can seriously jeopardize the patient's health.

Risks for the patient's health have also been found in the case of positive displacement.

A further large drawback of known needle-free connectors is represented by the presence of clearance between the different pieces from which they are made. In fact, such clearance can jeopardize the isolation inside the connector or in any case contribute to those internal pressure variations which produce the above-mentioned negative or positive displacement.

Special types of needle-free connectors are disclosed by the patent documents US 5,676,346, WO 2006/122406 and US 2009/209922. The document WO 97/21463 discloses all the features of the preamble of claim 1.

### Description of the Invention

The main aim of the present invention is to provide a needle-free connector for infusion lines which permits avoiding or strongly restricting the above-mentioned negative or positive displacements.

A further object of the invention is to provide a needle-free connector for infusion lines, in which the possibility of any clearance between its component parts is reduced to the utmost.

Another object of the present invention is to provide a needle-free connector for infusion lines which can overcome the above mentioned drawbacks of the prior art in the ambit of a simple, rational, easy and effective to use as well as low cost solution.

The above mentioned objects are achieved by the present needle-free connector for infusion lines made according to claim 1.

Moreover, the above mentioned objects are achieved by the method for manufacturing a needle-free connector for an infusion line, made according to claim 4.

### Brief Description of the Drawings

Other characteristics and advantages of the present invention will become better evident from the description of a preferred, but not exclusive, embodiment of a needle-free connector for infusion lines, illustrated by way of an indicative, but not limitative example in the accompanying drawings in which:
Figure 1 is an axonometric view of the connector according to the invention;
Figure 2 is an axonometric view of a manufacturing step of the connector according to the invention;
Figure 3 is a longitudinal section view of the connector according to the invention, before the deformation step of the manufacturing step;
Figure 4 is the view of Figure 3 in a next step;

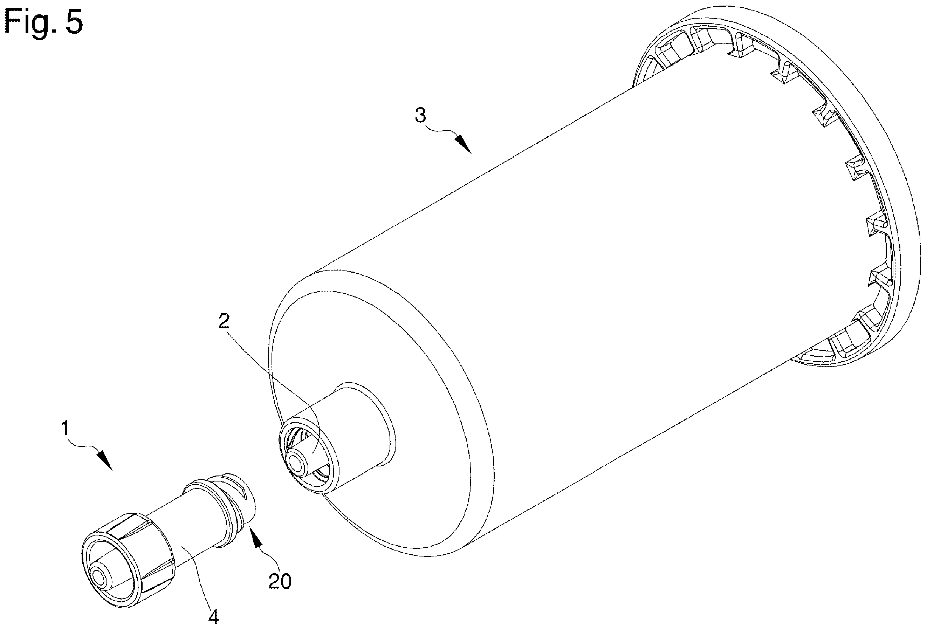

Figure 5 is an axonometric view of the connector and of a syringe before their coupling;

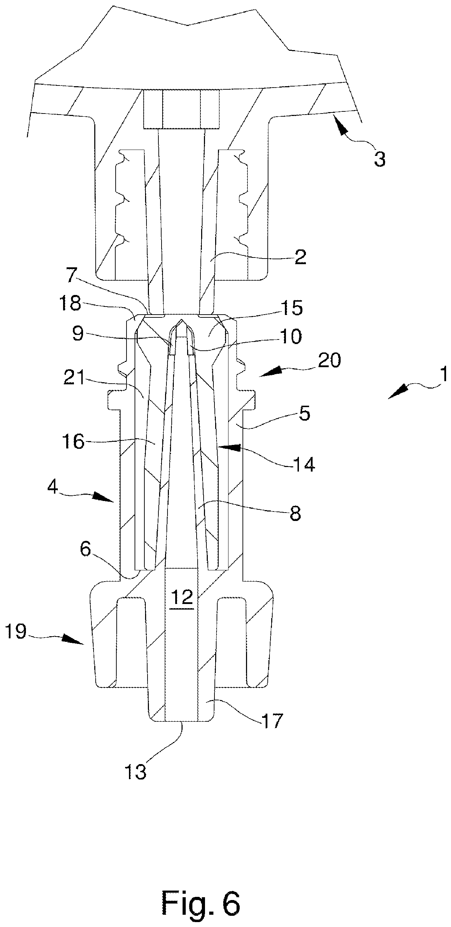

Figure 6 is a longitudinal section view of the connector according to the invention which is moved close to the syringe; and
Figure 7 is the view of the previous illustration, wherein the connector is coupled to the syringe.

### Embodiments of the Invention

With reference to these figures, globally indicated by 1 is the needle-free connector of the invention.

The proposed connector 1 is intended for use with an infusion line, especially of the type comprising an intravenous catheter or of the type made up of or comprising a medical bag or the like.

In practice, the connector is an inlet valve for the administration of medical fluids or saline solution, etc.

In detail, the connector 1 is of the type adapted to receive the outlet sleeve 2 (or *taper*) of the needle-free syringe 3 containing the above-mentioned fluid.

The connector 1 comprises a containment body 4 which can be coupled, preferably in a releasable way, both to said infusion line and to said syringe 3.

Preferably, this occurs by using locking systems of the Luer lock type or the like.

In detail, at its distal end 20, intended for coupling to the syringe 3, and at its proximal end 19, intended for coupling to the catheter, the connector 1 has threaded coupling means that can be of the Luer lock type or the like.

Herein below, for more convenient exposition but without loss of general information, reference will be made to the case wherein the infusion line is made up of the above-mentioned catheter, connected at one end to a vessel of a patient while at the other end it is coupled to the connector 1.

In practice, as is known, the patient's circulatory system remains connected to the catheter, while the syringe 3 is coupled to the connector 1 only when a substance has to be administered.

At the end of administration, the syringe 3 is separated from the connector 1. The containment body 4 of connector 1 is hollow and comprises an entry 7 to its internal volume.

The containment body 4 is preferably made of polycarbonate or other plastic material.

The body 4 comprises a housing 5, of tubular shape, intended to be coupled to the syringe 3, in the way explained above.

The housing 5 has a base 6, at its own proximal end, and an entry 7 at the opposite distal end 11, i.e. at the free end.

The connector 1 of the invention comprises a pipe 8, 17 suitable for the fluid-dynamic communication with the catheter, following coupling between this and the connector 1.

In practice, the pipe 8, 17 places the inside of the body 4 in communication with the catheter.

In turn, the pipe 8, 17 comprises a tubular element 8, substantially needle shaped, having at least an access 9, 10 and protruding from the base 6 into the housing 5, to define between the tubular element 8 and the housing 5 itself a chamber, which is closed at the base 6.

Furthermore, the pipe 8, 17 comprises an outlet tang 17, communicating with the tubular element 8, suitable for being coupled sealed to the catheter. Preferably, as clearly shown in the illustrations, tang 17 and tubular element 8 are coaxial.

In practice, the pipe 8, 17 of the invention is separated into two parts by the above base 6 to define the tubular element 8 and the tang 17.

The tubular element 8 therefore protrudes from the base 6 towards the inside of the housing 5, so that between the external side surface thereof and the internal lateral surface of the housing 5 a chamber is identified which is closed at the base 6.

Preferably, the housing 5 and the pipe 8, 17 are axial-symmetric, coaxial and both elongated.

The tubular element 8 is shorter than the housing 5 and its free end 11 does not protrude from the above-mentioned entry 7 and is in fact separate from this and located inside the housing 5.

Always preferably, the tubular element 8 is tapered towards its free end 11, which can be pointed.

Even more in detail, the tubular element 8 comprises one or more access through holes 9, 10 obtained on its side wall, in order to define a fluid-dynamic path 12 which originates from said holes 9, 10, crosses the tubular element 8 and the tang 17 and terminates in the exit 13 of the latter intended, during use, to be located within the catheter.

In practice, said fluid-dynamic path is defined by an inner channel 12 obtained axially to the connector 1, which channel 12 crosses the length of the pipe 8, 17.

Preferably, the body 4 of connector 1 is, in its entirety, axial-symmetric.

The connector 1 comprises a sealing element 14, this too preferably axial-symmetric, contained in the housing 5.

The sealing element 14 is suitable for moving between an occlusion position wherein it seals the entry 7 of the housing 5, preventing the fluid-dynamic communication between the outside of the connector 1 and the aforementioned fluid-dynamic path 12, and an opening position wherein it frees the entry 7.

The sealing element 14 is fitted sealed over the tubular element 8.

In detail, in its occlusion position, the sealing element 14 obstructs the access 9, 10 of the tubular element 8.

The connector 1 is constituted by only two members made up of the containment body 4 and of the sealing element 14, each of which is made in a single body piece.

This way, the clearance is eliminated or drastically reduced between the different parts of the invention and the drawbacks of prior art are overcome.

The housing 5 shapes, at its distal end 11, an inwardly folded portion 18 so as to define the aforementioned entry 7.

The housing 5 is tubular and, in this case, the folded portion 18 has an annular shape to define centrally the above-mentioned entry 7 of the body 4 of the connector 1.

In particular, the housing 5 can be substantially cylindrical.

In this case, the entry 7 has smaller dimensions than the inner section of the housing 5 which can also be constant along its entire length.

For the purpose of making a connector 1 which is made up of only two pieces but which also comprises such folded portion 18, the invention envisages a manufacturing method which comprises the steps explained below.

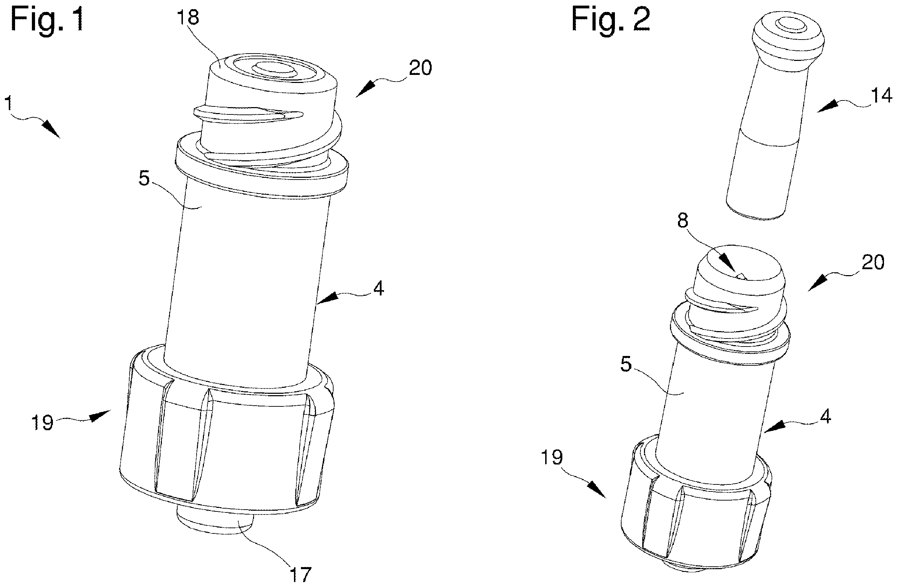

First of all, the containment body 4 of the connector 1 is provided, comprising the housing 5 described above and the sealing element 14 (see figure 2) is provided.

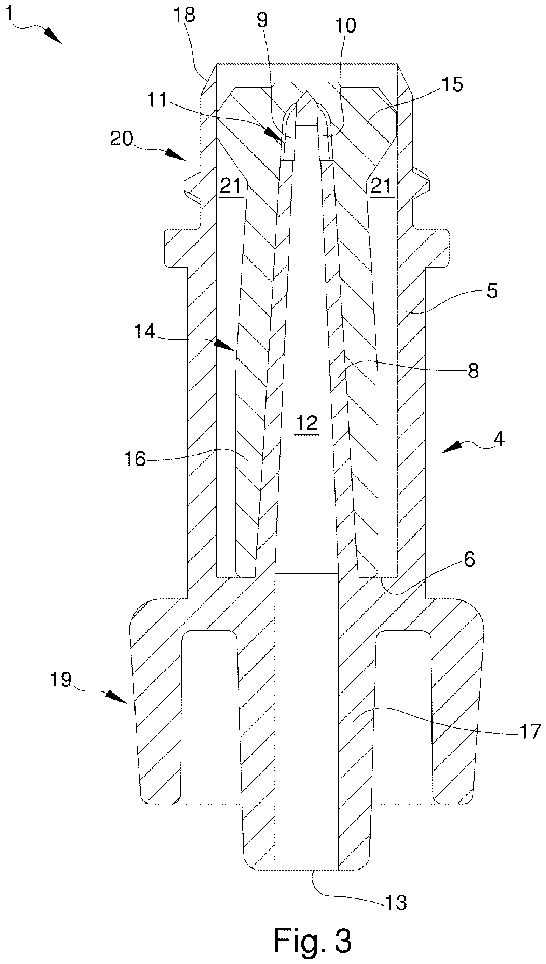

The sealing element 14 is inserted in the housing 5 so the tubular element 8 fits into the sealing element 14 itself, to obtain the reciprocal configurations already explained in the next paragraphs (see figure 3).

After which, a portion of the housing 5, comprising the above open and distal end 11, is heated so as to soften it.

Such heating can e.g. be done by means of the use of ultra-sounds.

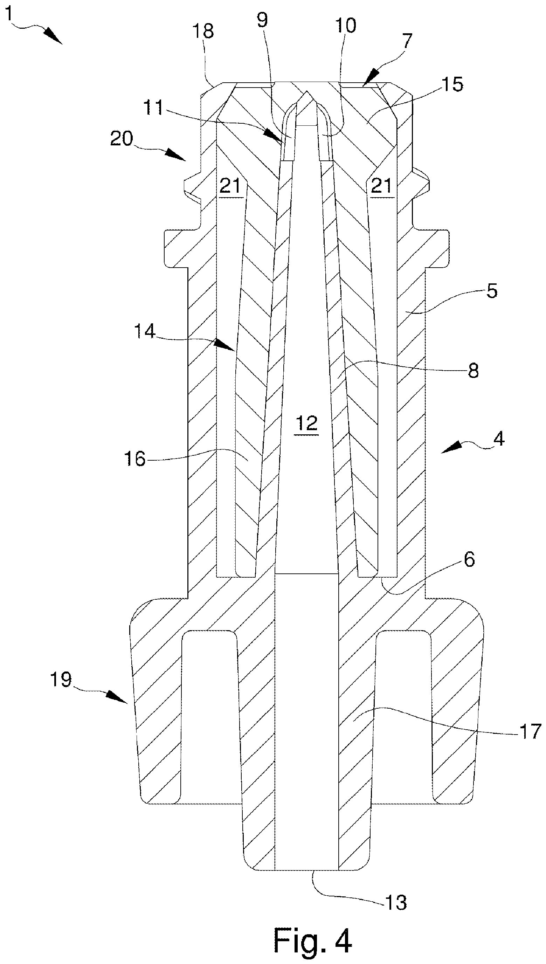

At this point, the heated portion is deformed to define the above folded portion 18 (see fig. 4).

Also note that the method of the invention can also envisage a cold deformation of the above extremal portion of the housing 5.

The sealing element 14 of the invention comprises an occlusion portion 15 which plugs the above-mentioned chamber, to define a pneumatic spring.

The chamber of the housing 5 comprises air and, since it is closed at the base 6, as explained above, and since it is plugged by the occlusion portion 15 of the sealing element 14, a closed space, or inter-space 21, is created in it, which provides resistance to the compression caused by the shift of the sealing element 14.

To be precise, such occlusion portion 15 completely occupies, instant by instant, a section of the chamber, i.e., one of its longitudinal portions.

At the time of describing the operation of the invention, it will be explained how such pneumatic spring helps achieve the proposed objects.

The sealing element 14 is flexible, and preferably made of a yielding and in particular compressible material, e.g., silicone.

In detail, the sealing element 14 is fitted tightly over the tubular element 8 and, completely covers it when it is in its occlusion position.

The sealing element comprises a sheath 14 which tightly covers the external surface of the tubular element 8.

In this case, the sealing element 14 can comprise a cavity, preferably blind, suitable for housing at least a distal portion of the tubular element 8 comprising its free end.

In practice, the sheath can internally shape a cavity, e.g., with a shape complementary to that of the tubular element 8, which cavity, when idle, can have smaller dimensions than those of such element.

This way, once the sealing element 14 has been fitted over the tubular element 8, the flexible characteristics of the former hold it firmly onto the latter.

In particular, the sheath is coupled with interference to the tubular element 8.

The sheath shapes a thickened head 15 comprising the above-mentioned occlusion portion.

In this case, the sealing element 14 can also comprise a thinner tail 16 than the head 15, shaped like a sleeve, which tail 16 extends between the head 15 and the base 6 of the housing 5.

In practice, the head 15 abuts with interference the internal lateral surface of the housing 5, and therefore of the chamber, and is suitable for sliding along said tubular element 8, without the continuous adhesion between sheath and tubular element 8 ever failing.

The above mentioned folded portion 18 abuts the head 15, crushing it, when it is in the above occlusion position.

Consequently, the pneumatic spring is also preloaded when it is in "idle" position, which is more precisely the position of maximum extension, which corresponds to the occlusion position of the sealing element 14.

This aspect not only contributes in general to the sealed isolation of the inside of connector 1, but in detail helps to ensure that the only space subject to change in pressure inside the connector 1 is always and only that placed outside the sheath, i.e., in the inter-space 21 defined around it.

The preload helps keep the sheath 14 adhered on the tubular element 8, because it experiments a pressure from the outside towards the inside which keeps it abutted with the surface of the tubular element 8.

The access 9, 10 or the accesses of the tubular element 8 are placed in distal portion of the tubular element 8, the head 15 of the sealing element 14 closes and seals, at the same time, such accesses and the entry 7 of the housing 5, when the sealing element 14 is in occlusion position.

In an embodiment not shown, the sheath 14, at one or more points along the above-mentioned tail 16, has a number of ribs or grooves to obtain its programmed deformation.

Said ribs or grooves can be obtained at the external surface (facing the inter-space 21) or the inner surface (i.e., in the above-mentioned inner cavity) of the sheath itself.

This solution facilitates both the collapse and the elastic return in the initial configuration of the sheath 14.

Herein below, for convenience of exposure and without loss of general information, reference will be made to the preferred case wherein the tubular element is a pointed needle 8 having a number of above-mentioned side holes arranged at its free distal end.

In this case, the head 15 of the sheath 14 makes up a distal end of the sealing element 14 through which the needle 8 or better its pointed distal end 11 can penetrate.

Consequently, when the sheath 14 is in the occlusion position, the head 15 surrounds and surmounts the tip of the needle 8, extending above it until it closes the access 9, 10, while it extends laterally between the needle 8 and the housing 5 to plug the chamber.

In detail, the distal portion of the needle 8 which comprises the accesses 9, 10 is completely contained in the head 15, when the sealing element 14 is in the occlusion position and the head 15 is therefore at the distal portion of the housing 5 abutted with its folded portion 18.

When the sealing element 14 moves towards the opening position, according to the procedures shown below, the head 15 moves towards the base 6 of the housing 5, allowing itself to be perforated by the tip of the needle 8 and surpassing the distal portion of the latter, so as to free the entry 7 of the housing 5 and the accesses of the needle 8, while however keeping sealed the above-mentioned inter-space 21 which helps define the pneumatic spring.

In practice, the elasticity characteristics of the head 15 are such that the needle 8 is able to perforate it through a deformable orifice formed in it, which orifice closes again automatically, sealing itself, when the sealing element 14 returns to the occlusion position and the needle 8 therefore exits from the channel.

While the head 15 is pierced by the needle 8, the side walls of the latter are always very adhered onto the orifice, so as to prevent any flow of fluids, including air, through the orifice itself.

It must be noticed that the fact that the needle 8 comprises a tip without openings at its top, which tip penetrates the head 15 of the sheath 14 also when this is in the occlusion position, permits avoiding having empty spaces or discontinuities between the needle 8 and the head 15, wherein air or other fluids could trickle, thus germinating pathogens and in any case potentially compromising the isolation of the fluid-dynamic path 12 from the non-sterilized external environment.

In an embodiment of the invention not shown here, the housing 5 comprises, at its base 6, at least a groove wherein the proximal end of the sheath 14 is interference fitted.

In practice, the edge of the proximal end, which preferably identifies a ring around the needle 8, is fastened within a groove obtained for the purpose in the base 6, so as to assist the tight adhesion of sheath 14 to the external surface of the needle 8.

The operation of the invention is described below.

Initially, the connector 1 is connected to the catheter at its proximal end 19, while its distal end 20 which comprises the entry 7 is free.

The entry 7 is closed by the sealing element 14 which, in this step, is in the occlusion position, where it isolates and seals the above fluid-dynamic path 12. Every time the medical staff members have to administer a substance, they couple the syringe 3 to the housing 5 causing its dispensing sleeve 2 to penetrate through the entry 7.

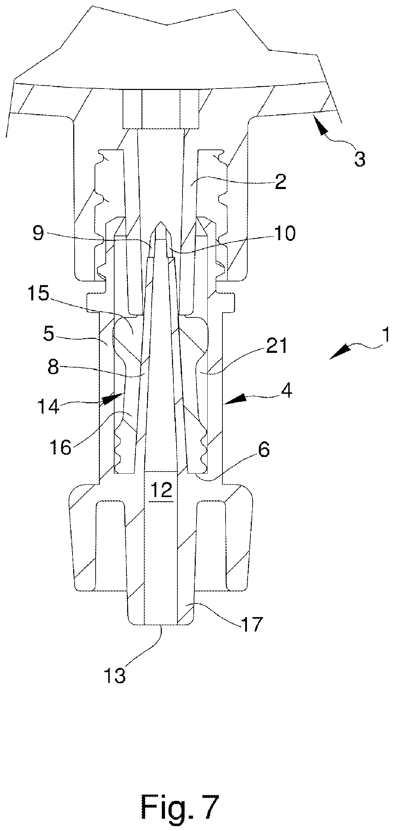

This way, the sleeve 2, or taper, of the syringe 3 presses against the head 15 of the sealing element 14, moving it along the needle 8 in the direction of the several times mentioned base 6 (see figure 7).

In this step, the distal portion of the needle 8 is uncovered, and the sleeve 2 of the syringe 3 receives it internally.

When the sealing element 14 reaches the opening position, the sleeve 2 of the syringe 3 also internally comprises the accesses 9, 10 of the needle 8.

The dimensions and the shape of needle 8 and sleeve 2 are selected in such a way that the former fits sealed into the latter.

In this step, the pneumatic spring is loaded, by effect of both the compression of the air contained in the above inter-space 21, and of the elasticity of the sealing element itself.

In sliding along the needle 8, the sheath 14 always remains substantially strongly adhered to it, including thanks to the increase in pressure inside the inter-space 21 which compresses the sheath 14 against the external surface of the needle 8.

This way, no empty space is created between sealing element 14 and needle 8 or between needle 8 and sleeve 2.

In this step, the fluid-dynamic path 12 defined within the connector 1 places the inside of the syringe 3 in communication with the catheter.

Consequently, the health worker injects the envisaged dose of fluid from the syringe 3 to the catheter passing through the connector 1.

At this point, the operator extracts the sleeve 2 from the housing 5 of the connector 1.

Advantageously, the sealing element 14 climbs back up along the needle 8, as the sleeve 2 is gradually removed from the latter, without delay and without therefore allowing an empty space to form between sleeve 2, needle 8 and sealing element 14, especially at the distal portion of the needle 8.

This way, when the sleeve 2 passes by the accesses, the fluid included in the fluid-dynamic path 12 does not undergo any appreciable aspiration in the direction of the accesses of the needle 8 and, consequently, no negative displacement is produced of the blood within the catheter, except to a negligible extent.

What is more, the invention completely prevents the occurrence of positive displacements.

## Claims

1. Needle-free connector (1) for an infusion line, comprising:
- a containment body (4) which can be coupled to said line, comprising at least an entry (7) and comprising at least an internal pipe (8, 17) suitable for the fluid-dynamic communication with said entry (7) and with said line; and
- a sealing element (14) suitable for moving between an occlusion position wherein it seals said entry (7) and an opening position wherein it frees said entry (7);
wherein:
- said body (4) shapes a housing (5) having a base (6) at one proximal end and said entry (7) at the opposite distal end (20);
- said body (4) shapes internally a tubular element (8) having at least an access (9, 10), which tubular element (8) is part of said pipe (8, 17),
- said tubular element (8) is arranged within said housing (5) and protrudes from said base (6) of the housing (5) so as to define an internal chamber closed at said base (6),
- said housing (5) shapes, at said distal end (20), an inwardly folded portion (18) so as to define said entry (7); and
- said housing (5) is tubular and said folded portion (18) has an annular shape and defines centrally said entry (7),
- said sealing element (14) is flexible and comprises a sheath (14) which tightly covers the external surface of said tubular element (8), and
said sealing element (14) comprises at least an occlusion portion (15) which plugs said chamber sealed, to define a pneumatic spring, and wherein the sheath (14) shapes a thickened head (15) which comprises said occlusion portion (15), which abuts the internal lateral surface of said housing (5) and which is suitable for sliding along said tubular element (8) **characterized in that** the needle-free connector (1) is constituted by only two members, which are said body (4) and said sealing element (14) each of which is made in a single body piece; and said folded portion (18) abuts said occlusion portion (15) when the latter is in said occlusion position so that said pneumatic spring is preloaded when said sealing element (14) is in its idle position, wherein said idle position involves the sealing member being at the position of maximum extension, which corresponds to said occlusion position.

2. Connector (1) according to claim 1 **characterized by** the fact that said body (4) is axial-symmetric.

3. Connector (1) according to claim 1 or 2, **characterized by** the fact that said housing (5) comprises, at said base (6), at least a groove wherein a proximal end of said sheath (14) is interference fitted.

4. Method to make a needle-free connector (1) for an infusion line, as defined in claims 1-3, wherein the method comprises the steps of:
- providing a containment body (4) made of plastic material, which can be coupled to said line, is made in a single body piece and comprises: at least a housing (5) having an open end, and at least an internal pipe (8, 17) suitable for the fluid-dynamic communication with said housing (5) and with said line;
- inserting completely a sealing element (14) in said housing (5) through said open end, said sealing element (14) being made in a single body piece; and
- after said inserting, deforming a portion of said housing (5) comprising said open end, so as to define an inwardly folded portion (18) that abuts the sealing element (14) and keeps the sealing element (14) preloaded,
wherein:
- said housing (5) has a base (6) at one proximal end and an entry (7) at the opposite distal end (20);
- said housing (5) shapes, at said distal end (20), said inwardly folded portion (18) so as to define said entry (7); and
- said housing (5) is tubular and said folded portion (18) has an annular shape and defines centrally said entry (7), and
- said connector (1) is constituted by only two members which are said body (4) and said sealing element (14), each of which is made in a single body piece.

5. Method according to claim 4, **characterized by** the fact that, before said deforming, said portion of the housing (5) is heated so as to soften it.

6. Method according to claim 5, **characterized by** the fact that said portion of the housing (5) is heated by means of the use of ultra-sounds.

## Patentansprüche

1. Nadelloser Verbinder (1) für eine Infusionsleitung, aufweisend:
- einen Einhausungskörper (4), der mit der Leitung gekoppelt werden kann, umfassend mindestens einen Eingang (7) und mindestens ein inneres Rohr (8, 17), das für die fluiddynamische Verbindung mit dem Eingang (7) und mit der Leitung geeignet ist; und
- ein Dichtungselement (14), das zum Bewegen zwischen einer Verschlussposition, in der es den Eingang (7) abdichtet, und einer Öffnungsposition, in der es den Eingang (7) freigibt, geeignet ist;
wobei:
- der Körper (4) ein Gehäuse (5) bildet, das eine Basis (6) an einem proximalen Ende und den Eingang (7) an dem gegenüberliegenden distalen Ende (20) aufweist;
- der Körper (4) innen ein rohrförmiges Element (8) mit mindestens einem Zugang (9, 10) ausbildet, wobei das rohrförmige Element (8) Teil des Rohres (8, 17) ist,
- das rohrförmige Element (8) innerhalb des Gehäuses (5) angeordnet ist und von der Basis (6) des Gehäuses (5) hervorsteht, um eine an der Basis (6) geschlossene innere Kammer zu definieren,
- das Gehäuse (5) an dem distalen Ende (20) einen nach innen abgekanteten Abschnitt (18) ausbildet, um den Eingang (7) zu definieren; und
- das Gehäuse (5) rohrförmig ist und der abgekantete Abschnitt (18) eine ringförmige Form aufweist und zentral den Eingang (7) definiert,
- das Dichtungselement (14) flexibel ist und eine Hülle (14) umfasst, welche die Außenfläche des rohrförmigen Elements (8) enganliegend überdeckt, und
- das Dichtungselement (14) mindestens einen Verschlussabschnitt (15) umfasst, der die abgedichtete Kammer verstöpselt, um eine pneumatische Feder zu definieren, und wobei die Hülle (14) einen verdickten Kopf (15) ausbildet, der den Verschlussabschnitt (15) umfasst, der an der inneren Seitenfläche des Gehäuses (5) anliegt und der zum Gleiten entlang des rohrförmigen Elements (8) geeignet ist;
**dadurch gekennzeichnet, dass**
der nadellose Verbinder (1) aus nur zwei Elementen besteht, welche der Körper (4) und das Dichtungselement (14) sind, von denen jedes in einem einstückigen Körper hergestellt ist; und
der abgekantete Abschnitt (18) an den Verschlussabschnitt (15) angrenzt, wenn dieser sich in der Verschlussposition befindet, sodass die pneumatische Feder vorgespannt ist, wenn sich das Dichtungselement (14) in seiner Ruheposition befindet, wobei die Ruheposition das Dichtungselement in der Position der maximalen Ausdehnung umfasst, die der Verschlussposition entspricht.

2. Verbinder (1) nach Anspruch 1, **dadurch gekennzeichnet, dass** der Körper (4) axialsymmetrisch ist.

3. Verbinder (1) nach Anspruch 1 oder 2, **dadurch gekennzeichnet, dass** das Gehäuse (5) an der Basis (6) mindestens eine Nut aufweist, in die ein proximales Ende der Hülle (14) in Presspassung eingepasst ist.

4. Verfahren zur Herstellung eines nadellosen Verbinders (1) für eine Infusionsleitung, wie in den Ansprüchen 1 bis 3 definiert, wobei das Verfahren die folgenden Schritte umfasst:
- Bereitstellen eines Einhausungskörpers (4), der aus Kunststoffmaterial hergestellt ist, der mit der Leitung gekoppelt werden kann, in einem einstückigen Körper hergestellt ist und umfasst: mindestens ein Gehäuse (5), das ein offenes Ende und mindestens ein inneres Rohr (8, 17) aufweist, das für die fluiddynamische Verbindung mit dem Gehäuse (5) und mit der Leitung geeignet ist;
- vollständiges Einsetzen eines Dichtungselements (14) in das Gehäuse (5) durch das offene Ende, wobei das Dichtungselement (14) aus einem einstückigen Körper hergestellt ist; und
- nach dem Einsetzen Verformen eines Abschnitts des Gehäuses (5), der das offene Ende umfasst, um einen nach innen abgekanteten Abschnitt (18) zu definieren, der an dem Dichtungselement (14) anliegt und das Dichtungselement (14) vorgespannt hält,
wobei:
- das Gehäuse (5) eine Basis (6) an einem proximalen Ende und einen Eingang (7) an dem gegenüberliegenden distalen Ende (20) aufweist;
- das Gehäuse (5) an dem distalen Ende (20) den nach innen abgekanteten Abschnitt (18) ausbildet, um den Eingang (7) zu definieren; und
- das Gehäuse (5) rohrförmig ist und der abgekantete Abschnitt (18) eine ringförmige Form aufweist und zentral den Eingang (7) definiert, und
- der Verbinder (1) aus nur zwei Elementen besteht, welche der Körper (4) und das Dichtungselement (14) sind, von denen jedes in einem einstückigen Körper hergestellt ist.

5. Verfahren nach Anspruch 4, **dadurch gekennzeichnet, dass** vor der Verformung der Abschnitt des Gehäuses (5) erwärmt wird, um ihn weicher zu machen.

6. Verfahren nach Anspruch 5, **dadurch gekennzeichnet, dass** der Abschnitt des Gehäuses (5) durch die Verwendung von Ultraschall erwärmt wird.

## Revendications

1. Connecteur sans aiguille (1) pour un tube de perfusion, comprenant :
- un corps de retenue (4) qui peut être couplé audit tube, comprenant au moins une entrée (7) et comprenant au moins un tuyau interne (8, 17) adapté pour la communication dynamique de fluide avec ladite entrée (7) et avec ledit tube ; et
- un élément d'étanchéité (14) adapté pour se déplacer entre une position d'occlusion dans laquelle il assure l'étanchéité de ladite entrée (7) et une position d'ouverture dans laquelle il libère ladite entrée (7) ;
dans lequel :
- ledit corps (4) forme un logement (5) ayant une base (6) au niveau d'une extrémité proximale et ladite entrée (7) au niveau de l'extrémité distale (20) opposée ;
- ledit corps (4) forme à l'intérieur un élément tubulaire (8) ayant au moins un accès (9, 10), lequel élément tubulaire (8) fait partie dudit tuyau (8, 17),
- ledit élément tubulaire (8) est agencé au sein dudit logement (5) et dépasse depuis ladite base (6) du logement (5) de façon à définir une chambre interne fermée au niveau de ladite base (6),
- ledit logement (5) forme, au niveau de ladite extrémité distale (20), une portion repliée vers l'intérieur (18) de façon à définir ladite entrée (7) ; et
- ledit logement (5) est tubulaire et ladite portion repliée (18) a une forme annulaire et définit au centre ladite entrée (7),
- ledit élément d'étanchéité (14) est flexible et comprend une gaine (14) qui couvre étroitement la surface externe dudit élément tubulaire (8), et
ledit élément d'étanchéité (14) comprend au moins une portion d'occlusion (15) qui bouche ladite chambre de façon étanche, pour définir un ressort pneumatique, et dans lequel la gaine (14) forme une tête épaissie (15) qui comprend ladite portion d'occlusion (15), qui bute contre la surface latérale interne dudit logement (5) et qui est adaptée pour coulisser le long dudit élément tubulaire (8)
**caractérisé en ce que**
le connecteur sans aiguille (1) est constitué uniquement de deux organes, qui sont ledit corps (4) et ledit élément d'étanchéité (14) dont chacun est réalisé en une pièce d'un seul tenant ; et
ladite portion repliée (18) bute contre ladite portion d'occlusion (15) lorsque cette dernière est dans ladite position d'occlusion de sorte que ledit ressort pneumatique soit préchargé lorsque ledit élément d'étanchéité (14) est dans sa position à l'arrêt, dans lequel ladite position à l'arrêt implique que l'organe d'étanchéité soit à la position d'extension maximale, qui correspond à ladite position d'occlusion.

2. Connecteur (1) selon la revendication 1, **caractérisé par le fait que** ledit corps (4) est axial symétrique.

3. Connecteur (1) selon la revendication 1 ou 2, **caractérisé par le fait que** ledit logement (5) comprend, au niveau de ladite base (6), au moins une gorge dans laquelle une extrémité proximale de ladite gaine (14) est ajustée avec serrage.

4. Procédé pour réaliser un connecteur sans aiguille (1) pour un tube de perfusion, tel que défini aux revendications 1 à 3, dans lequel le procédé comprend les étapes de :
- fourniture d'un corps de retenue (4) réalisé en matériau plastique, qui peut être couplé audit tube, est réalisé en une pièce d'un seul tenant et comprenant : au moins un logement (5) ayant une extrémité ouverte, et au moins un tuyau interne (8, 17) adapté pour la communication dynamique de fluide avec ledit logement (5) et avec ledit tube ;
- insertion complète d'un élément d'étanchéité (14) dans ledit logement (5) à travers ladite extrémité ouverte, ledit élément d'étanchéité (14) étant réalisé en une pièce d'un seul tenant ; et
- après ladite insertion, déformation d'une portion dudit logement (5) comprenant ladite extrémité ouverte, de façon à définir une portion repliée vers l'intérieur (18) qui bute contre l'élément d'étanchéité (14) et garde l'élément d'étanchéité (14) préchargé,
dans lequel :
- ledit logement (5) a une base (6) au niveau d'une extrémité proximale et une entrée (7) au niveau de l'extrémité distale (20) opposée ;
- ledit logement (5) forme, au niveau de ladite extrémité distale (20), ladite portion repliée vers l'intérieur (18) de façon à définir ladite entrée (7) ; et
- ledit logement (5) est tubulaire et ladite portion repliée (18) a une forme annulaire et définit au centre ladite entrée (7), et
- ledit connecteur (1) est constitué uniquement de deux organes qui sont ledit corps (4) et ledit élément d'étanchéité (14), dont chacun est réalisé en une pièce d'un seul tenant.

5. Procédé selon la revendication 4, **caractérisé par le fait que**, avant ladite déformation, ladite portion du logement (5) est chauffée pour la ramollir.

6. Procédé selon la revendication 5, **caractérisé par le fait que** ladite portion du logement (5) est chauffée par l'utilisation d'ultrasons.
